(19) Europäisches Patentamt
European Patent Office
Office européen des brevets

(11) Numéro de publication: **0 054 488**
**B1**

# (12) FASCICULE DE BREVET EUROPEEN

(45) Date de publication du fascicule du brevet: **15.02.84**

(51) Int. Cl.³: **C 07 C 43/30**, H 01 B 3/36 // C07C41/52

(21) Numéro de dépôt: **81401981.6**

(22) Date de dépôt: **11.12.81**

**(54) Utilisation comme huile isolante de di(alkylphénoxy) méthanes et nouveaux di(alkylphénoxy) méthanes utilisables pour cette application.**

(30) Priorité: **11.12.80 FR 8026309**

(43) Date de publication de la demande: **23.06.82 Bulletin 82/25**

(45) Mention de la délivrance du brevet: **15.02.84 Bulletin 84/7**

(84) Etats contractants désignés: **CH DE GB LI SE**

(56) Documents cités:
**JOURNAL OF THE AMERICAN CHEMICAL SOCIETY, vol. 73, avril 1951 (US) S. MIRON et al.: "Bis-(substituted phenoxy)-methanes" pages 1872-1873**
**JOURNAL OF THE AMERICAN CHEMICAL SOCIETY, vol. 72, novembre 1950 (US) E. MONROE et al.: "Some Bis-(aryloxy)-methanes" pages 5345-5346**
**CHEMICAL ABSTRACTS, vol. 90, no. 19, 7 mai 1979 ref.: 151805a, page 575 COLUMBUS, OHIO (US)**
**C.R. ACAD. SC. PARIS, tome 269, 22 décembre 1969, série C (FR) C. PIGENET et al.: "Sur les conformations des bis-(phénoxy)-, tris-(phénoxy)- et tétra-kis-(phénoxy)-méthanes et de leurs correspondants sulfurés" pages 1587, 1589**

(73) Titulaire: **COMPAGNIE FRANCAISE DE RAFFINAGE Société anonyme dite:, 5, rue Michel-Ange, F-75781 Paris Cedex 16 (FR)**

(72) Inventeur: **Marty, Claude, 66, rue Guillemard, F-76600 Le Havre (FR)**
Inventeur: **Engelhard, Philippe, 197, rue Félix Faure, F-76600 Le Havre (FR)**

(74) Mandataire: **Jolly, Jean-Pierre et al, Cabinet BROT 83, rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 054 488 B1

Utilisation comme huile isolante de di(alkylphénoxy) méthanes et nouveaux di(alkylphénoxy) méthanes utilisables pour cette application.

La présente invention concerne l'utilisation comme diélectriques liquides d'une famille de diphénoxyméthanes, dont chaque noyau aromatique est substiué par au moins un reste alkyle possédant trois atomes de carbone ou plus, et dont certains sont des composés nouveaux.

On sait que les huiles isolantes utilisées dans les appareillages électriques doivent posséder, outre leurs nécessaires propriétés diélectriques (rigidité et permittivité élevée, ainsi que des pertes faibles), un certain nombre de qualités propres à leurs conditions d'emploi:

- une faible viscosité jusqu'aux basses températures,
- un point d'écoulement suffisamment bas pour permettre une bonne répartition de l'huile sous des conditions climatiques variées,
- un point d'éclair élevé qui soit supérieur à 150°C, et une bonne résistance à l'inflammation,
- une bonne capacité d'absorption des gaz, en particulier vis-à-vis de l'hydrogéne, de façon à garantir des qualités d'isolation constantes,
- une grande stabilité dans le temps, ainsi que vis-à-vis des agents oxydants,
- un prix compétitif et, par conséquent, une structure chimique facilement accessible par les moyens classiques,
- enfin, selon des critères qui prennent une importance croissante, une toxicité faible, ainsi qu'une biodégradabilité élevée.

La plupart des composés qui entrent dans les compositions diélectriques liquides utilisées jusqu'à présent appartiennent essentiellement au groupe des huiles naphténiques issues de coupes pétrolières et à celui des polyphényles chlorés.

L'ensemble des propriétés de ces deux types d'huile n'est cependant que l'expression d'un compromis, et l'une ou l'autre des caractéristiques souhaitées fait toujours défaut. De nombreuses recherches ont donc été lancées pour remplacer les huiles naphténiques, dont l'approvisionnement devient plus difficile, et pour remplacer les polyphényles chlorés, à cause de leur toxicité et de leur très faible biodégradabilité.

Un certain nombre de solutions ont été recherchées, par exemple en remplaçant les atomes de chlore des polychlorophényles par des restes alkyle (brevet américain n° 2 977 554 et demande de brevet français n° 2 335 920), puis en cherchant des structures nouvelles telles que celles des éthers de thiophényle (brevet américain n° 3 235 605) ou celles des phtalates de dialkyle (demande de brevet français n° 2 362 474). Mais il faut reconnaître que ces tentatives n'ont pas abouti au développement escompté, car l'une ou l'autre des qualités essentielles précédemment citées fait habituellement défaut.

La Demanderesse a maintenant découvert qu'il est possible d'obtenir, à partir d'une matière première simple et peu coûteuse, des composés non chlorés, dont certains sont connus et qui ont des priopriétés les rendant aptes à remplacer avantageusement un certain nombre d'huiles utilisées jusqu'à présent dans le domaine de l'isolation électrique, sans en présenter les inconvénients.

Les composés utilisables selon la présente invention sont les di-(alkylphénoxy) méthanes représentés par la formule générale I

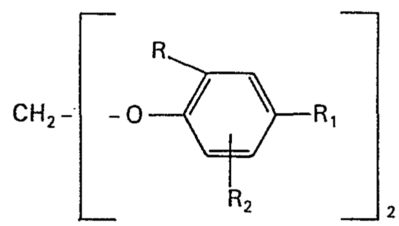

(I)

dans laquelle R, $R_1$ et $R_2$ peuvent représenter un atome d'hydrogène ou un reste alkyle, l'un au moins des restes R, $R_1$ ou $R_2$ possédant entre trois et dix atomes de carbone. Les composés dialkylés de cette formule sont, à la connaissance de la Demanderesse, des composés nouveaux, qui constituent un autre objet de la présente invention.

De même que leurs analogues inférieurs, qui sont utilisables dans les compositions odorantes et qui sont décrits dans la demande de brevet français n° 2 391 984, le composés de la présente invention peuvent être préparés par réaction de l'alkylphénol correspondant avec un halogénure de méthylène, notamment le chlorure de méthylène, en présence d'un hydroxyde alcalin ou alcalino-terreux et d'un catalyseur de transfert de phase. Cette réaction est illustrée par le schéma réactionnel suivant:

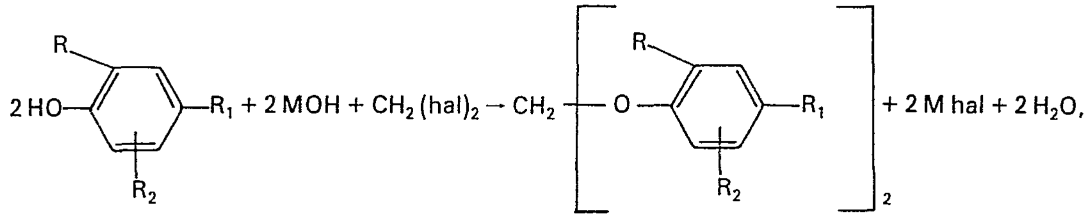

où M représente un métal alcalin ou alcalino-terreux et hal est un halogène.

Parmi les phénols substitués, ou leurs mélanges, utilisables comme produits de départ de la réaction, il convient d'utiliser les composés dont l'un au moins des substituants est un reste

alkyle linéaire ou ramifié possédant 3 à 10 atomes de carbone et, de préférence, entre 3 et 6. On a, en effet, observé qu'une chaîne carbonée trop courte ou trop longue peut avoir une influence néfaste sur la viscosité des composés I obtenus. Les restes alkyles préférés, dans l'invention, sont donc les restes n- ou iso-propyle, n-, iso- ou tertio-butyle, n- ou isopentyle, n- ou iso-hexyle, méthyl-2 n-pentyle, n- ou isoheptyle, sans pour autant que cette liste soit limitative.

De nombreux solvants inertes tels que le benzène, le tétrahydrofurane, le diméthylsufoxyde ou l'hexaméthyl phosphore triamide, sont susceptibles d'être utilisés pour effectuer la présente réaction, mais il s'avère, dans la plupart des cas, plus simple d'utiliser le chlorure de méthylène lui-même en simple excès.

Les bases de départ utilisables pour effectuer cette réaction sont les hydroxydes de métaux alcalins ou alcalino terreux. On utilise donc le plus couramment la potasse et la soude. Les conditions de température varient de 0 à 100°C, suivant les cas, en fonction du solvant et du catalyseur choisi.

Les catalyseurs préférés selon l'invention sont les catalyseurs dits «de transfert de phase», qui permettent d'atteindre, dans des conditions opératoires très douces, des réactivités élevées, en particulier dans les cas de réactions nucléophiles. Parmi ces catalyseurs, les halogénures d'ammonium ou de phosphonium semblent les plus appropriés, ainsi que les éthers de polyglycols, les polyéthers ou les «éthers couronnes».

Une variante de préparation des composés I consiste à utiliser, comme produits de départ des mélanges d'alkylphénols mono-, di- ou trisubstitués, et à les faire réagir selon le mode opératoire précédemment décrit. Les mélanges de di-(alkylphénoxy) méthanes I, qui sont ainsi directement obtenus, peuvent être soit directement utilisés dans les compositions d'huiles isolantes, soit séparés par distillation pour obtenir plusieurs produits purs.

Les di-(alkylphénoxy) méthanes I utilisables selon la présente invention, ainsi que leurs mélanges, se distinguent par une gamme de propriétés, qui les rend particulièrement adaptés à une utilisation comme huile isolante:

- de même que leur point d'ébullition, leur point d'éclair est élevé,
- grâce à l'absence de chlore et sans doute leur fonction éther, leur biodégradabilité est supérieure à celles des polychlorophényles,
- leur synthèse s'effectue dans des conditions de procédés simples, ce qui permet de valoriser de nombreux alkylphénols ou mélanges d'alkylphénols.

De ce fiat, les composés I ou les mélanges de composés I selon la présente invention peuvent être utilisés dans des compositions diélectriques, soit seuls, soit en mélange avec d'autres huiles isolantes.

Il est également possible, par un choix judicieux des composants de tels mélanges diélectriques, d'obtenir une gamme de produits possédant l'ensemble des propriétés précédemment énoncées, en les réglant, éventuellement à l'aide d'adjuvants, en fonction du type de transformateur auquel la composition diélectrique est destinée. On peut ainsi leur adjoindre un certain nombre d'adjuvants usuels tels que des agents abaisseurs de point d'écoulement, choisis, par exemple, parmi les polymères.

Les exemples suivants visent à illustrer plus précisément l'invention, sans toutefois la limiter.

Exemple 1
Préparation du di-(4-sec.butylphénoxy) méthane.

Ce composé répond à la formule I ci-dessus, dans laquelle $R_2 = R = H$, et $R_1 = \overset{CH_3}{\overset{|}{C}}H - CH_2 - CH_3$.

Dans un réacteur de 6 litres, on introduit 1000 g (6,6 moles) de 4-sec.butylphénol. On ajoute ensuite, par petites portions et sous agitation, 855 g (15,3 moles) de potasse solide.

Après addition de la potasse, on ajoute, goutte à goutte, sous agitation, un mélange contenant 216 g de bromure de tétrabutylammonium et 1400 g (16,5 moles) de chlorure de méthylène.

Le mélange réactionnel est ensuite maintenu pendant 15 heures, sous agitation, à une température comprise entre 25 et 35°C, puis versé dans 1500 cm³ d'eau. La phase organique est alors séparée et la phase aqueuse extraite trois fois par 100 cm³ de chlorure de méthylène.

Après réunion de la phase organique et du solvant d'extraction, on procède à un lavage avec une solution d'acide chlorhydrique 6N, puis à l'eau jusqu'à pH neutre.

Après séchage et évaporation du chlorure de méthylène, on fractionne le résidu sous pression réduite. On isole ainsi 1000 g de di-(4-sec.butylphénoxy)méthane:

- rendement observé, par rapport au rendement théorique, voisin de 97%,
- point d'ébullition voisin de 400°C,
- pureté chromatographique (CPV): 98%,
- identification effectuée sans ambiguïté par infrarouge et spectrométrie de masse:

IR: Bandes caractéristiques
bande éther C—O—C à 1210 $cm^{-1}$
bande C=C du noyau aromatique à 1600 $cm^{-1}$
substituant alkyle en position para sur le noyau

para 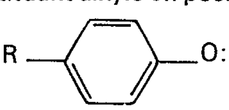

bande au voisinage de 830 $cm^{-1}$.
SM: (spectre de masse haute résolution)
pic moléculaire:
312,2102 $C_{21}H_{28}O_2$ (théorie 312,2089)
pics fragments:

283,1698 $C_{19}H_{23}O_2$ ($C_4H_9 \cdot C_6H_4 \cdot O\,CH_2O \cdot$

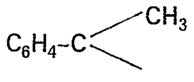
)

163,1123 $C_{11}H_{15}O$ ($C_4H_9 \cdot C_6H_4 \cdot OCH_2-$)
133,1017 $C_{10}H_{13}$ ($C_4H_9 \cdot C_6H_4-$)

Exemple 2
Synthèse du di-(2,6-diisopropylphénoxy) méthane.
Ce composé répond à la formule I, dans laquelle $R=R_2=CH\,(CH_3)_2$ et $R_1=H$.

Dans les mêmes conditions que celles de l'exemple 1, on ajoute 855 g (15,3 moles) de potasse solide à 1175 g (6,6 moles) de 2,6-diisopropylphénol.

On ajoute ensuite, goutte à goutte, un mélange de 230 g de bromure de tétrabutylammonium et de 1400 g (16,5 moles) de chlorure de méthylène.

Après la réaction, les produits sont séparés et permettent d'isoler 1190,2 g de di-(2,6-diisopropylphénoxy) méthane, qui est un composé nouveau:
- point d'ébullition: voisin de 400°-410°C,
- pureté chromatographique: 98%,
- rendement voisin de 98%.

Exemple 3
Synthèse du di-(3-isopropylphénoxy) méthane.
La formule de ce composé est la formule I, avec $R_2=CH\,(CH_3)_2$ et $R=R_1=H$.

Utilisant le même appareillage que dans l'exemple 1, on introduit 897,6 g (6,6 moles) de 3-isopropylphénol. On ajoute ensuite, par petites portions et sous agitation, 855 g (15,3 moles) de potasse solide.

Après addition de 197 g de chlorure de tétrabutyl phosphonium et 1400 g (16,5 moles) de chlorure de méthylène, le mélange réactionnel est maintenu sous agitation pendant 6 heures entre 60 et 80°C. Après réaction, le traitement est identique à celui de l'exemple 1.

Par fractionnement, on isole 917,3 g de di-(3-isopropylphénoxy) méthane:
- point d'ébullition: 340°C,
- pureté chromatographique: 98%,
- rendement voisin de 97%.

Exemple 4 à 9
En reprenant les conditions opératoires des exemples 1 à 3, les composés suivants ont été préparés avec des rendements sensiblement quantitatifs:

Exemple 4
Le di-(2-isopropylphénoxy) méthane,
dont le point d'ébullition se situe aux alentours de 320–330°C.

Exemple 5
Le di-(4-teramylphénoxy) méthane,
dont le point d'ébullition se situe aux environs de 440°C.

Exemple 6
Le di-(2,5-diisopropylphénoxy) méthane,
qui est un composé nouveau, dont le point d'ébullition se situe à environ 390–400°C.

Exemple 7
Le di-(3,5-diisopropylphénoxy) méthane,
qui est un composé nouveau, dont le point d'ébullition se trouve entre 395 et 405°C.

Exemple 8
Le di-(2,4-diméthyl-6-ter.butylphénoxy) méthane,
qui est un composé nouveau, dont le point d'ébullition se situe aux alentours de 420 à 430°C.

Exemple 9
Le di-(2,4,6-triisopropylphénoxy) méthane,
qui est un composé nouveau, possédant un point d'ébullition supérieur à 420°C.

Exemple 10
Synthèse de mélanges de di-(alkylphénoxy) méthanes.

De la même façon que dans l'exemple 1, on introduit un mélange d'alkylphénols, comprenant:
- 333 g (2,2 moles) de 4-sec.butylphénol,
- 391 g (2,2 moles) de 2,6 diisopropylphénol,
- 391 g (2,2 moles) de 2,4-diméthyl-6-ter.butylphénol.

On ajoute ensuite, par petites portions et sous agitation, 855 g (15,3 moles) de potasse solide. Après addition de 211 g de chlorure de triphénylméthylphosphonium et de 1400 g (16,5 moles) de chlorure de méthylène, le mélange réactionnel est maintenu sous agitation pendant 6 heures entre 60 et 80°C. Après réaction, on effectue un traitement identique à celui de l'exemple 1.

Le fractionnement donne 1048 g du mélange de di-(alkylphénoxy) méthanes, ce qui correspond à un rendement à peu près quantitatif par rapport à la quantité de phénols de départ.

Cette huile peut être utilisée telle quelle comme huile isolante. Par distillation fractionnée, elle permet également d'isoler les composés à l'état pur.

Exemple 11
Utilisation comme huile isolante.

Les caractéristiques diélectriques complètes du di-(4-sec.butylphénoxy) méthane préparé selon l'exemple 1 ont été étudiées et comparées à celles de deux huiles appartenant à chacune des grandes familles d'huiles isolantes couramment utilisées jusqu'à présent.

Les résultats de ces comparaisons figurent au Tableau 1.

Ils mettent en évidence des propriétés diélectriques et physiques du di-(4-sec.butylphénoxy) méthane en moyenne supérieures ou égales aux diverses caractéristiques des autres huiles. Cette supériorité est particulièrement visible en ce qui concerne la rigidité diélectrique.

Tableau 1
Comparaison des caractéristiques:

| | Di-(4-sec.butyl-phénoxy) méthane | Huile d'origine naphténique | Huile de type polyphényle chloré |
|---|---|---|---|
| Pertes diélectriques | $6 \cdot 10^{-4}$ | $2 \cdot 10^{-4}$ | $2 \cdot 10^{-2}$ |
| Permittivité relative | 2,8 | 2,3 | 4,6 |
| Rigidité diélectrique (kV) | 72,5 | 60,5 | 49,5 |
| Viscosité à 20°C | 86,6 | 36,5 | |
| Viscosité à 50°C (cst) | 15,0 | 11,0 | |
| Densité à 15°C | 0,997 | 0,89 | 1,56 |
| Point Eclair Cleveland | 196°C | 160°C | Néant |
| Point d'écoulement | −33°C | −25°C | −32°C |
| Dépôts après tests d'oxydation % poids | <0,01 | 0,07 | |

Exemple 12
Compositions de di-(alkylphénoxy) méthanes utilisables comme huiles isolantes.

Au cours de cette expérience, on a comparé la permittivité relative et la rigidité diélectrique d'une composition comprenant:

- 50% en poids de di-(4-sec.butylphénoxy) méthane.
- 50% en poids d'huile minérale d'origine naphténique,

à la permittivité et la rigidité de cette huile naphténique seule, d'une part, et à celles du di-(4-sec.butylphénoxy) méthane seul, d'autre part.

Les résultats de cette comparaison figurent dans le Tableau 2 et mettent nettement en évidence une amélioration sensible des propriétés diélectriques de la composition, notamment en ce qui concerne la rigidité diélectrique.

Tableau 2

| | Huile d'origine naphténique | Composition 50%–50% | Di-(4-sec.butyl-phénoxy)méthane |
|---|---|---|---|
| Permittivité relative | 2,3 | 2,5 | 2,8 |
| Rigidité diélectrique (kV) | 60,5 | 71 | 72,5 |

**Revendications**

1. Utilisation comme fluide diélectrique, dans des compositions d'huiles isolantes, éventuellement en mélange avec d'autres fluides diélectriques connus en soi, de di-(alkylphénoxy) méthanes de formule

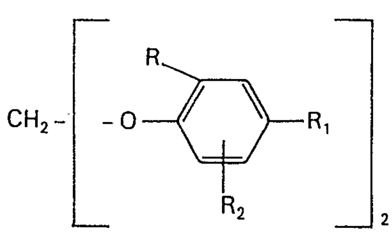

(I)

dans laquelle R, $R_1$ et $R_2$ représentent un atome d'hydrogène ou un reste alkyle, l'un au moins de ces substituants étant un reste alkyle linéaire ou ramifié possédant entre trois et dix atomes de carbone.

2. Utilisation selon la revendication 1, dans laquelle le composé de formule (I) est le di-(4-sec.butylphénoxy) méthane.

3. Utilisation selon la revendication 1, dans laquelle le composé de formule (I) est le di-(2,5-diisopropylphénoxy) méthane, le di-(2,6-diisopropylphénoxy) méthane ou le di-(3,5-diisopropylphénoxy) méthane.

4. Utilisation selon la revendication 1, dans laquelle le composé de formule (I) est le di-(2-isopropylphénoxy) méthane ou de di-(3-isopropylphénoxy) méthane.

5. Utilisation selon la revendication 1, dans laquelle le composé de formule (I) est le di-(4-ter.amylphénoxy) méthane.

6. Utilisation selon la revendication 1, dans laquelle le composé de formule (I) est le di-(2,4-diméthyl-6-ter.-butylphénoxy) méthane.

7. Utilisation selon la revendication 1, dans laquelle le composé de formule (I) est le di-(2,4,6-triisopropylphénoxy) méthane.

8. Les di-(alkylphénoxy) méthanes de formule

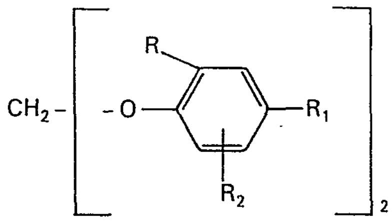

(I bis)

dans laquelle R, $R_1$ et $R_2$ représentent un atome d'hydrogène ou un reste alkyle, deux au moins de ces substituants étant un reste alkyle linéaire ou ramifié possédant entre trois et dix atomes de carbone.

9. Le di-(2,5-diisopropylphénoxy) méthane, le di-(2,6-diisopropylphénoxy) méthane et le di-(3,5-diisopropylphénoxy) méthane.

10. Le di-(2,4-diméthyl-6-ter.butylphénoxy) méthane.

11. Le di-(2,4,6-triisopropylphénoxy) méthane.

## Patentansprüche

1. Verwendung von Di-(alkylphenoxy)-methanen der Formel

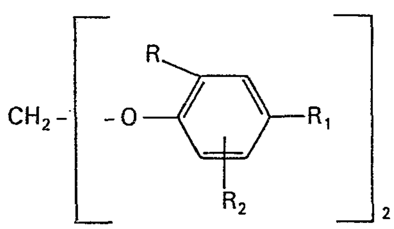

(I)

wobei R, $R_1$ und $R_2$ ein Wasserstoffatom oder einen Alkylrest darstellen und mindestens einer dieser Substituenten ein linearer oder verzweigter Alkylrest ist, welcher zwischen 3 und 10 Kohlenstoffatome aufweist; welche diese Verbindungen enthalten, als dielektrisches Strömungsmittel in Isolierölkompositionen, gegebenenfalls in Mischung mit anderen, an sich bekannten dielektrischen Strömungsmitteln.

2. Verwendung nach Anspruch 1, dadurch gekennzeichnet, dass es sich bei der Verbindung gemäss Formel (I) um Di-(4-sec.butylphenoxy)-methan handelt.

3. Verwendung nach Anspruch 1, dadurch gekennzeichnet, dass es sich bei der Verbindung gemäss Formel (I) um Di-(2,5-diisopropylphenoxy)-methan, Di-(2,6-diisopropylphenoxy)-methan oder Di-(3,5-diisopropylphenoxy)-methan handelt.

4. Verwendung nach Anspruch 1, dadurch gekennzeichnet, dass es sich bei der Verbindung gemäss Formel (I) um Di-(2-isopropylphenoxy)-methan oder Di-(3-isopropylphenoxy)-methan handelt.

5. Verwendung nach Anspruch 1, dadurch gekennzeichnet, dass es sich bei der Verbindung gemäss Formel (I) um Di-(4-ter.amylphenoxy)-methanhandelt.

6. Verwendung nach Anspruch 1, dadurch gekennzeichnet, dass es sich bei der Verbindung gemäss Formel (I) um Di-(2,4-dimethyl-6-ter.butylphenoxy)-methan handelt.

7. Verwendung nach Anspruch 1, dadurch gekennzeichnet, dass es sich bei der Verbindung gemäss Formel (I) um Di-(2,4,6-triisopropylphenoxy)-methan handelt.

8. Di-(alkylphenoxy)-methane der Formel

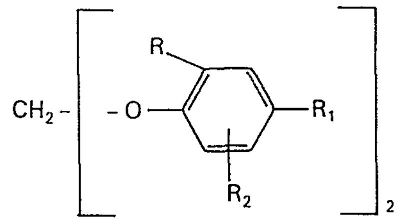

(I bis)

wobei R, $R_1$ und $R_2$ ein Wasserstoffatom oder einen Alkylrest darstellen und mindestens zwei dieser Substituenten ein linearer oder verzweigter Alkylrest sind, welcher zwischen 3 und 10 Kohlenstoffatome aufweist.

9. Di-(2,5-diisopropylphenoxy)-methan, Di-(2,6-diisopropylphenoxy)-methan und Di-(3,5-diisopropylphenoxy)-methan.

10. Di-(2,4-dimethyl-6-ter.butylphenoxy)-methan.

11. Di-(2,4,6-triisopropylphenoxy)-methan.

## Claims

1.Use as dielectric fluid, in insulating oil compositions, possibly mixed with other dielectric fluids known per se, of di-(alkylphenoxy) methanes of formule

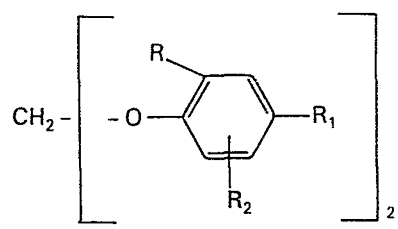

(I)

in which R, $R_1$ and $R_2$ represent a hydrogen atom or an alkyl remainder, one at least of these substituents being a linear or branched alkyl remainder having between three and ten carbon atoms.

2. The use according to claim 1, in which the compound of formula(1) is di-(4-sec.butylphenoxy) methane.

3. The use according to claim 1, wherein the compound of formula (1) is di-(2,5-diisopropylphenoxy) methane, di-(2,6-diisopropylphenoxy) methane or di-(3,5-diisopropylphenoxy) methane.

4. The use according to claim 1, wherein the compound of formula (1) is di-(2-isopropylphenoxy) methane or di-(3-isopropylphenoxy) methane.

5. The use according to claim 1 wherein the compound of formula (1) is di-(4-ter.amylphenoxy) methane.

6. The use according to claim 1, wherein the compound of formula (1) is di-(2,4-dimethyl-6-ter.-butylphenoxy) methane.

7. The use according to claim 1, wherein the compound of formula (1) is di-(2,4,6-triisopropylphenoxy) methane.

8. The di-(alkylphenoxy) methanes of formula

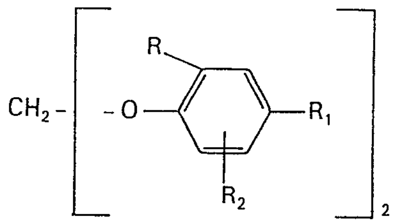

(I)

in which R, $R_1$ and $R_2$ represent a hydrogen atom or an alkyl remainder, two at least of these substituents being a linear or branched alkyl remainder having between three and ten carbon atoms.

9. Di-(2,5-diisopropylphenoxy) methane, di-(2,6 diisopropylphenoxy) methane and di-(3,5-diisopropylphenoxy) methane.

10. Di-(2,4-dimethyl-6-ter.butylphenoxy) methane.

11. Di-(2,4,6-triisopropylphenoxy) methane.